# EUROPEAN PATENT APPLICATION

(11) **EP 4 686 487 A1**
(43) Date of publication of application: **04.02.2026**
(21) Application number: 24207739.4
(22) Date of filing: 21.10.2024
(51) Int. Cl.: A61M 5/20, A61M 5/315, A61M 5/178

(54) **TUBULAR INJECTION DEVICE AND DRIVING MECHANISM**

(30) Priority: 31.07.2024 US 202463677420 P; 18.10.2024 US 202418919446
(71) Applicant: Altek Biotechnology Corporation, 300 Hsinchu City (TW)
(72) Inventor: HUANG, Yu-Cheng, 300 Hsinchu City (TW)
(74) Representative: Straus, Alexander

(57) **Abstract**

A tubular injection device (1, 2, 3, 5) with a proximal end (E1) and a distal end (E2) includes a drug storage member (10), an injection member (12), a movable member (14) and a driving mechanism (16). The injection member (12) is disposed at the proximal end (E1) and in fluid communication with the drug storage member (10). The movable member (14) is movably disposed in the drug storage member (10). The driving mechanism (16) includes a driving member (160), a transmission sleeve (162), a transmission rod (164) and a constraint member (166). The driving member (160) is disposed in the distal end (E2). The transmission sleeve (162) is engaged with the driving member (160). The transmission sleeve (162) includes an internal thread (1620). The transmission rod (164) includes an external thread (1640) meshing with the internal thread (1620). The constraint member (166) restrains the transmission rod (164) from rotating.

## Description

### Field of the Invention

The present invention relates to a tubular injection device and a driving mechanism, particularly a tubular injection device and a driving mechanism capable of improving injection operation and ensuring injection safety.

### Background of the Invention

Current injectors (e.g. auto-injectors or pen injectors) are used for self-administration of small quantity drugs in a single-dose or multiple-dose manner. Most existing injectors are pure mechanical-based, and are driven by mechanical push against a plunger rod of a cartridge or syringe, respectively. Furthermore, in order to transfer a medical fluid from a vial to the injector, users need to hold the vial with respect to the injector and pull the plunger rod of the injector by manual operation. The manual operation is not much easy for common users and injection safety may be influenced due to misoperation.

### Summary of the Invention

The present invention aims at providing a tubular injection device and a driving mechanism capable of improving injection operation and ensuring injection safety, thereby resolving the aforesaid problems.

This is achieved by a tubular injection device according to claim 1 and a driving mechanism according to claim 11. The dependent claims pertain to corresponding further developments and improvements.

As will be seen more clearly from the detailed description following below, the claimed tubular injection device with a proximal end and a distal end includes a drug storage member, an injection member, a movable member and a driving mechanism. The injection member is disposed at the proximal end and in fluid communication with the drug storage member. The movable member is movably disposed in the drug storage member. The driving mechanism includes a driving member, a transmission sleeve, a transmission rod and a constraint member. The driving member is disposed in the distal end. The transmission sleeve is engaged with the driving member. The transmission sleeve includes an internal thread. The transmission rod is disposed in the transmission sleeve and engaged with the movable member. The transmission rod includes an external thread meshing with the internal thread of the transmission sleeve. The constraint member restrains the transmission rod from rotating. The driving member drives the transmission sleeve to rotate, the transmission sleeve drives the transmission rod to move by an interaction between the internal thread and the external thread, and the transmission rod drives the movable member to move with respect to the drug storage member.

In an embodiment, when the driving member drives the transmission sleeve to rotate in a first direction, the transmission rod moves linearly toward the proximal end and drives the movable member to move toward the injection member; when the driving member drives the transmission sleeve to rotate in a second direction, the transmission rod drives the movable member to move away from the injection member; the first direction is opposite to the second direction.

In an embodiment, the transmission rod has a first limit plane, the constraint member is formed with a through hole, the through hole has a second limit plane, an end of the transmission rod passes through the through hole, and the constraint member restrains the transmission rod from rotating by the first limit plane and the second limit plane.

In an embodiment, the driving mechanism further includes a screw nut embedded in the transmission sleeve, and the internal thread is provided by the screw nut.

In an embodiment, the tubular injection device further includes a first shell, wherein the drug storage member is accommodated in the first shell.

In an embodiment, the tubular injection device further includes a second shell, wherein the first shell and the second shell are assembled to accommodate the drug storage member and the driving mechanism, the constraint member includes a flange, and the flange is sandwiched between the first shell and the second shell.

In an embodiment, the tubular injection device further includes a second shell and a third shell, wherein the first shell and the second shell are assembled to accommodate the drug storage member, the transmission sleeve, the transmission rod and the constraint member, the constraint member includes a flange, the flange is sandwiched between the first shell and the second shell, the third shell is detachably connected to the second shell, the driving member is accommodated in the third shell, and the driving member includes a transmission portion detachably connected to the transmission sleeve.

In an embodiment, the tubular injection device further includes a second shell, wherein the driving mechanism is accommodated in the second shell, the second shell is detachably connected to the first shell, and the transmission rod is detachably connected to the movable member.

In an embodiment, the tubular injection device further includes an adaptor configured to be assembled to the first shell, wherein, when a drug container is loaded in the adaptor, the injection member penetrates into the drug container.

In an embodiment, the injection member, the drug storage member, the movable member and the driving mechanism are arranged along a longitudinal axis of the tubular injection device to form a handheld injector.

As will be seen more clearly from the detailed description following below, the claimed driving mechanism includes a driving member, a transmission sleeve, a transmission rod and a constraint member. The transmission sleeve is engaged with the driving member. The transmission sleeve includes an internal thread. The transmission rod is disposed in the transmission sleeve and engaged with the movable member. The transmission rod includes an external thread meshing with the internal thread of the transmission sleeve. The constraint member restrains the transmission rod from rotating. The driving member drives the transmission sleeve to rotate, and the transmission sleeve drives the transmission rod to move by an interaction between the internal thread and the external thread.

In an embodiment, when the driving member drives the transmission sleeve to rotate in a first direction, the transmission sleeve drives the transmission rod to move out of the transmission sleeve; when the driving member drives the transmission sleeve to rotate in a second direction, the transmission sleeve drives the transmission rod to move into the transmission sleeve; the first direction is opposite to the second direction.

In an embodiment, the transmission rod has a first limit plane, the constraint member is formed with a through hole, the through hole has a second limit plane, an end of the transmission rod passes through the through hole, and the constraint member restrains the transmission rod from rotating by the first limit plane and the second limit plane.

In an embodiment, the driving mechanism further includes a screw nut embedded in the transmission sleeve, and the internal thread is provided by the screw nut.

In an embodiment, the driving member includes a transmission portion detachably engaged with the transmission sleeve.

### Brief Description of the Drawings

In the following, the invention is further illustrated by way of example, taking reference to the accompanying drawings thereof:
FIG. 1 is a perspective view illustrating a tubular injection device according to an embodiment of the invention,
FIG. 2 is a sectional view illustrating the tubular injection device,
FIG. 3 is a sectional view illustrating a movable member moving toward an injection member,
FIG. 4 is a perspective view illustrating a driving mechanism,
FIG. 5 is an exploded view illustrating the driving mechanism,
FIG. 6 is a sectional view illustrating a tubular injection device according to an embodiment of the invention,
FIG. 7 is an exploded sectional view illustrating the tubular injection device,
FIG. 8 is an exploded view illustrating a driving mechanism,
FIG. 9 is a sectional view illustrating a tubular injection device according to an embodiment of the invention,
FIG. 10 is an exploded sectional view illustrating the tubular injection device,
FIG. 11 is a sectional view illustrating a tubular injection device according to an embodiment of the invention,
FIG. 12 is a sectional view illustrating a tubular injection device according to an embodiment of the invention, and
FIG. 13 is a sectional view illustrating a tubular injection device according to an embodiment of the invention.

### Detailed Description

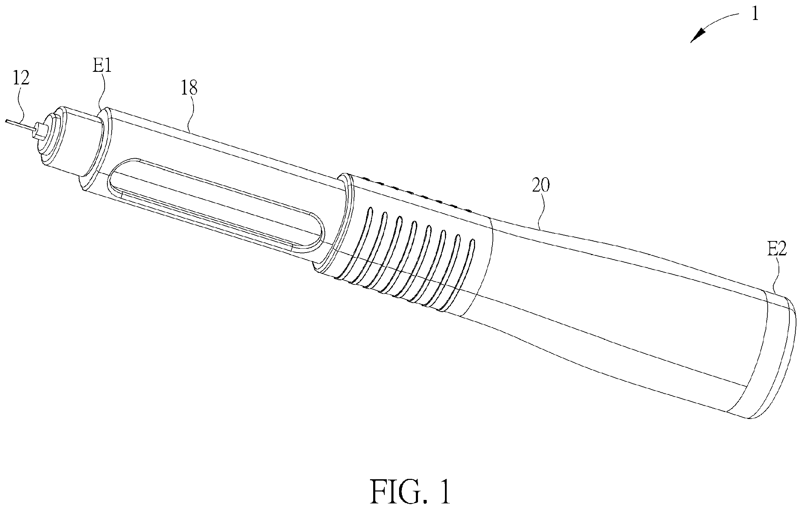

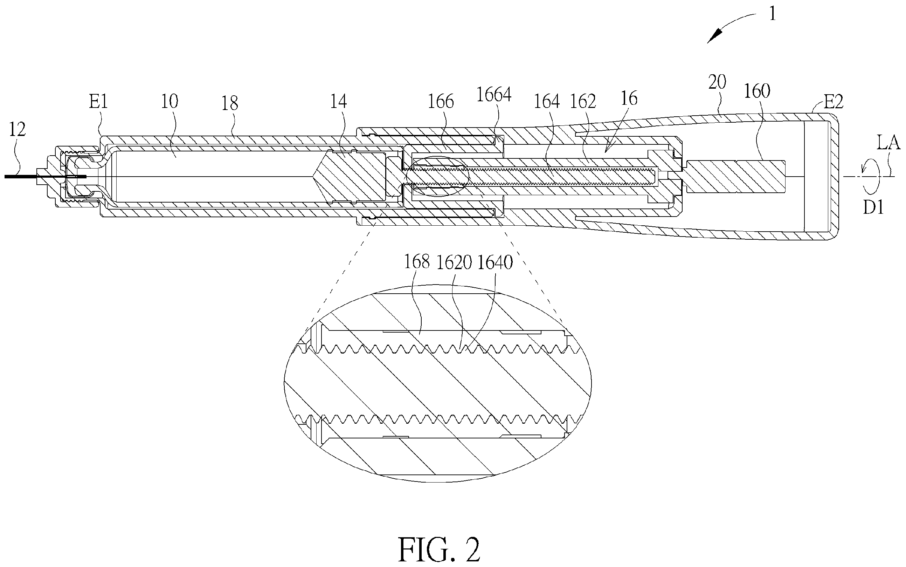

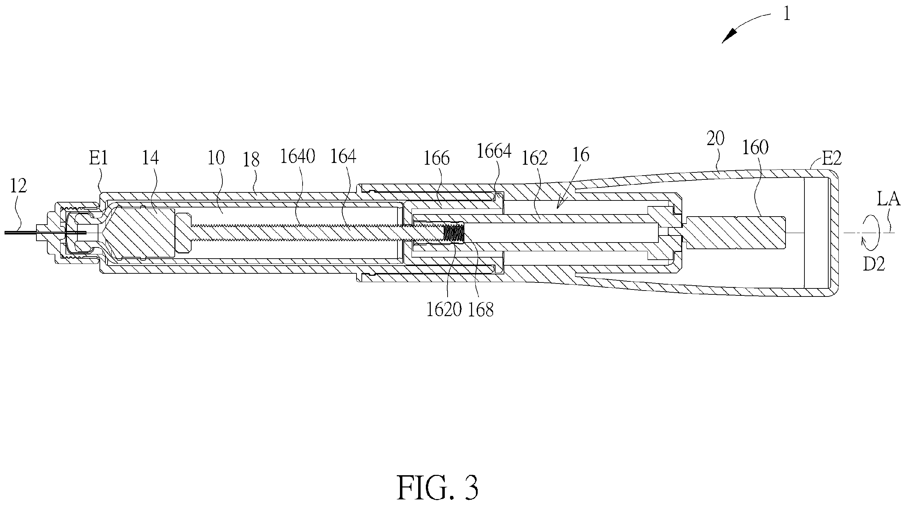

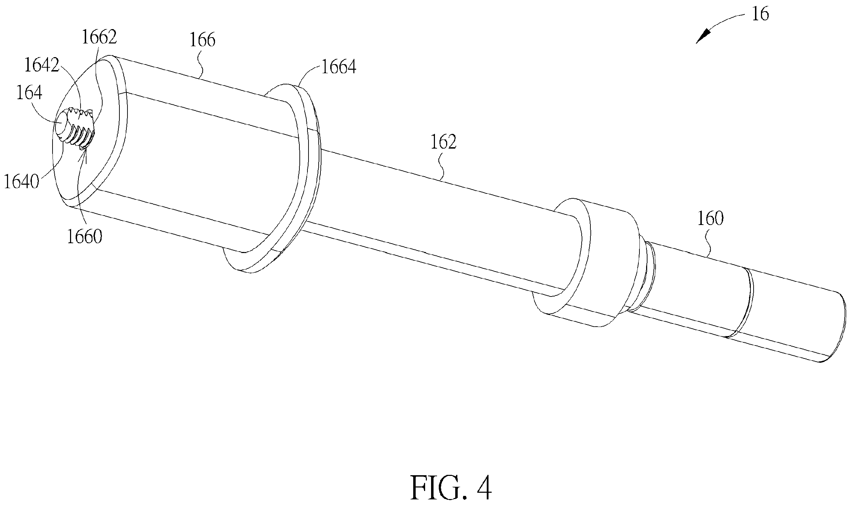

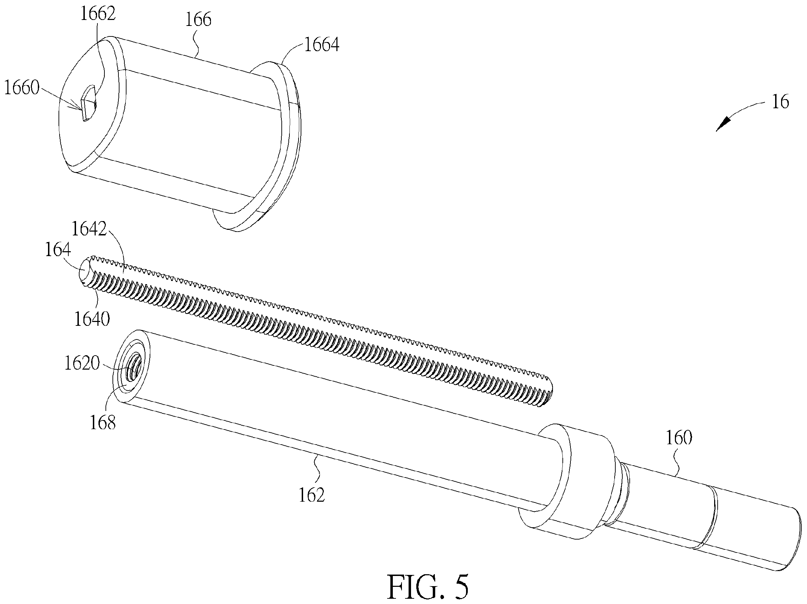

Referring to FIGs. 1 to 5, FIG. 1 is a perspective view illustrating a tubular injection device 1 according to an embodiment of the invention, FIG. 2 is a sectional view illustrating the tubular injection device 1, FIG. 3 is a sectional view illustrating a movable member 14 moving toward an injection member 12, FIG. 4 is a perspective view illustrating a driving mechanism 16, and FIG. 5 is an exploded view illustrating the driving mechanism 16.

As shown in FIGs. 1 to 3, the tubular injection device 1 with a proximal end E1 and a distal end E2 comprises a drug storage member 10, an injection member 12, a movable member 14, a driving mechanism 16, a first shell 18 and a second shell 20. The first shell 18 and the second shell 20 are assembled to accommodate the drug storage member 10, the movable member 14 and the driving mechanism 16. In other words, the drug storage member 10, the movable member 14 and the driving mechanism 16 are accommodated in a chamber formed by the first shell 18 and the second shell 20. In this embodiment, the injection member 12, the drug storage member 10, the movable member 14 and the driving mechanism 16 may be arranged along a longitudinal axis LA of the tubular injection device 1 to form a handheld injector, such that a user may hold the tubular injection device 1 to perform an injection process easily.

The injection member 12 is disposed at the proximal end E1 of the tubular injection device 1 and in fluid communication with the drug storage member 10. The movable member 14 is movably disposed in the drug storage member 10. In this embodiment, the injection member 12 may be an injection needle or the like, and the drug storage member 10 may be a syringe, a cartridge or the like. For example, the drug storage member 10 may be a cartridge, and the injection member 12 may be a pen needle detachably disposed on the proximal end E1 of the tubular injection device 1. The movable member 14 may be, but is not limited to, a plunger.

The driving mechanism 16 comprises a driving member 160, a transmission sleeve 162, a transmission rod 164 and a constraint member 166. The driving member 160 is disposed in the distal end E2 of the tubular injection device 1. In this embodiment, the driving member 160 may be a motor or the like. The transmission sleeve 162 is engaged with the driving member 160, such that the driving member 160 can drive the transmission sleeve 162 to rotate. The transmission rod 164 is disposed in the transmission sleeve 162 and engaged with the movable member 14. Furthermore, the transmission sleeve 162 comprises an internal thread 1620, and the transmission rod 164 comprises an external thread 1640 meshing with the internal thread 1620 of the transmission sleeve 162. Thus, the transmission sleeve 162 can drive the transmission rod 164 to move by an interaction between the internal thread 1620 and the external thread 1640, and the transmission rod 164 can drive the movable member 14 to move with respect to the drug storage member 10.

In this embodiment, the driving mechanism 16 may further comprise a screw nut 168 embedded in the transmission sleeve 162, and the internal thread 1620 may be provided by the screw nut 168. However, the invention is not so limited. For example, in another embodiment, the internal thread 1620 may be formed on an inner wall of the transmission sleeve 162, such that the screw nut 168 may be omitted. When the internal thread 1620 is formed on the inner wall of the transmission sleeve 162, the length of the internal thread 1620 may be smaller than or equal to the length of the transmission sleeve 162 and it depends on practical applications.

The constraint member 166 is configured to restrain the transmission rod 164 from rotating. In this embodiment, the transmission rod 164 may have a first limit plane 1642, the constraint member 166 may be formed with a through hole 1660, and the through hole 1660 may have a second limit plane 1662, as shown in FIGs. 4 and 5. An end of the transmission rod 164 passes through the through hole 1660 of the constraint member 166, such that the constraint member 166 can restrain the transmission rod 164 from rotating by the first limit plane 1642 and the second limit plane 1662. In this embodiment, the constraint member 166 may comprise a flange 1664. As shown in FIGs. 2 and 3, the flange 1664 may be sandwiched between the first shell 18 and the second shell 20, so as to restrain the constraint member 166 from rotating and moving. However, the invention is not so limited. For example, in another embodiment, the constraint member 166 may be fixed by engagement or other mechanical fixing manners, such that the flange 1664 may be omitted.

As shown in FIG. 3, when the driving member 160 drives the transmission sleeve 162 to rotate in a first direction D1 (e.g. an output shaft of the driving member 160 rotates counterclockwise), the transmission rod 164 may be driven by the transmission sleeve 162 to move linearly toward the proximal end E1 of the tubular injection device 1 and then drives the movable member 14 to move toward the injection member 12. As shown in FIG. 2, when the driving member 160 drives the transmission sleeve 162 to rotate in a second direction D2 (e.g. the output shaft of the driving member 160 rotates clockwise), the transmission rod 164 may be driven by the transmission sleeve 162 to move linearly away from the proximal end E1 of the tubular injection device 1 and then drives the movable member 14 to move away from the injection member 12. The first direction D1 is opposite to the second direction D2. That is to say, the first direction D1 may be counterclockwise and the second direction D2 may be clockwise or, alternatively, the first direction D1 may be clockwise and the second direction D2 may be counterclockwise. Accordingly, the tubular injection device 1 of the invention utilizes the driving mechanism 16 to drive the movable member 14 to move with respect to the drug storage member 10, so as to achieve automated injection of drug. Thus, users do not need to pull or push the movable member 14 to move by manual operation.

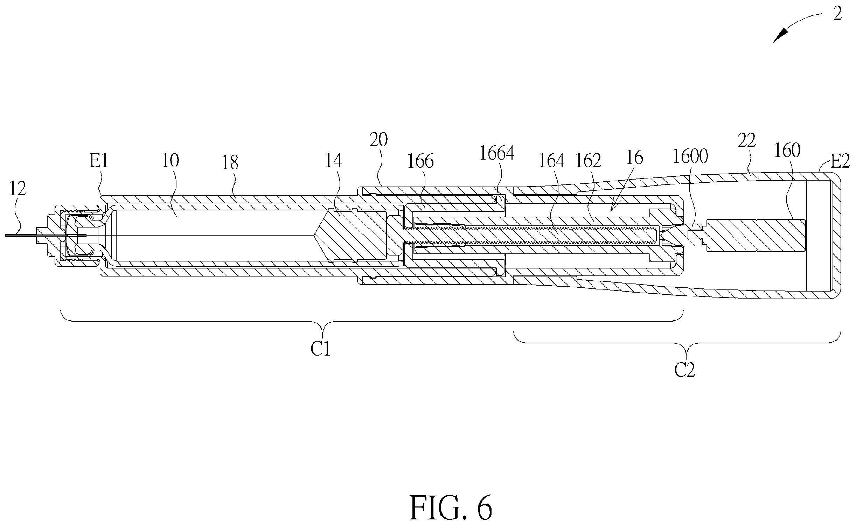

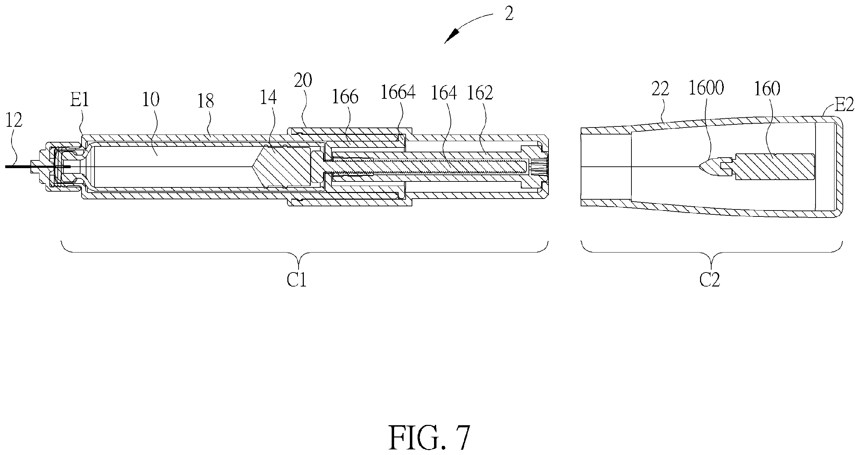

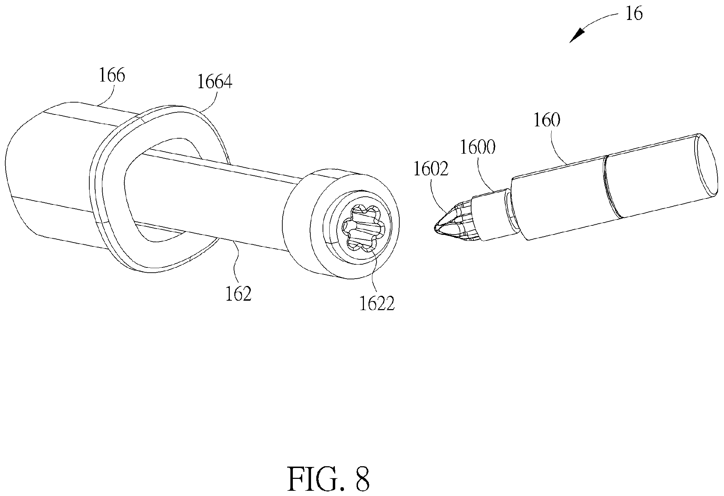

Referring to FIGs. 6 to 8, FIG. 6 is a sectional view illustrating a tubular injection device 2 according to an embodiment of the invention, FIG. 7 is an exploded sectional view illustrating the tubular injection device 2, FIG. 8 is an exploded view illustrating a driving mechanism 16.

The main difference between the tubular injection device 2 and the aforesaid tubular injection device 1 is that the tubular injection device 2 further comprises a third shell 22, as shown in FIGs. 6 and 7. In this embodiment, the first shell 18 and the second shell 20 are assembled to accommodate the drug storage member 10, the movable member 14, the transmission sleeve 162, the transmission rod 164 and the constraint member 166, wherein the flange 1664 of the constraint member 166 is also sandwiched between the first shell 18 and the second shell 20. The driving member 160 is accommodated in the third shell 22. The third shell 22 is detachably connected to the second shell 20, and the driving member 160 comprises a transmission portion 1600 detachably connected to the transmission sleeve 162. As shown in FIG. 8, the transmission portion 1600 may have a first engaging structure 1602 and the transmission sleeve 162 may have a second engaging structure 1622, such that the transmission portion 1600 may be detachably connected to the transmission sleeve 162 by engaging the first engaging structure 1602 with the second engaging structure 1622. Thus, the third shell 22 with the driving member 160 may be detached from the second shell 20 after injection, as shown in FIG. 7.

In this embodiment, the first shell 18 and the second shell 20 may be assembled to form a first chamber C1 for accommodating the drug storage member 10, the movable member 14, the transmission sleeve 162, the transmission rod 164 and the constraint member 166, and the third shell 22 may form a second chamber C2 for accommodating the driving member 160. Thus, the second chamber C2 may be detached from the first chamber C1 after injection, as shown in FIG. 7. The first chamber C1 with related components may be disposable after injection, whereas the second chamber C2 with related components may be disposable or reusable after injection.

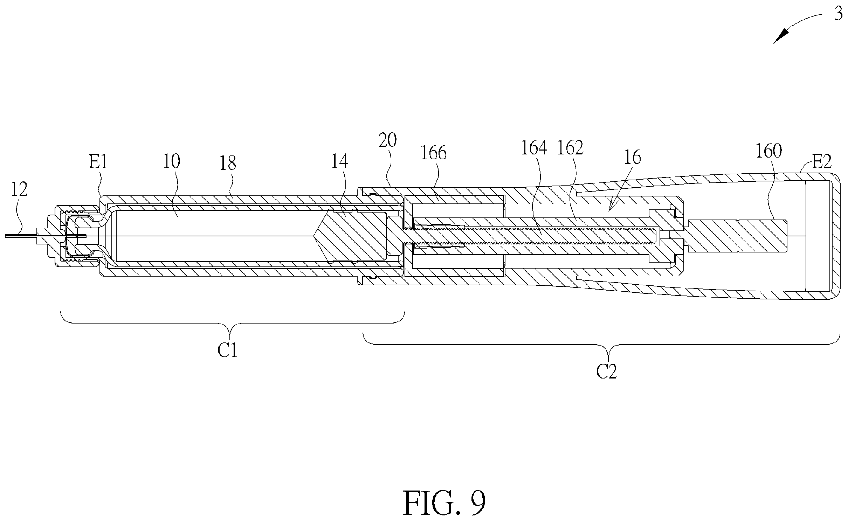

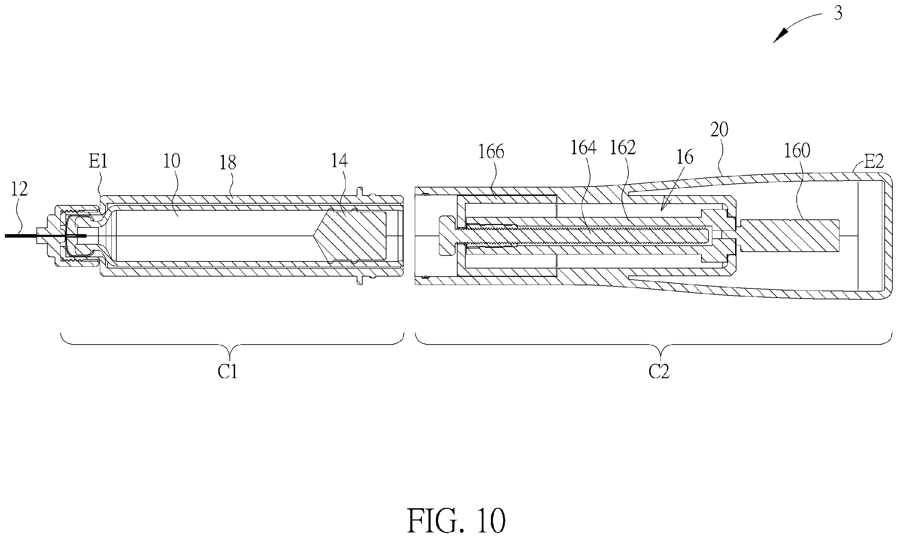

Referring to FIGs. 9 and 10, FIG. 9 is a sectional view illustrating a tubular injection device 3 according to an embodiment of the invention and FIG. 10 is an exploded sectional view illustrating the tubular injection device 3.

The main difference between the tubular injection device 3 and the aforesaid tubular injection device 1 is that the driving mechanism 16 of the tubular injection device 3 is accommodated in the second shell 20, as shown in FIGs. 9 and 10. In this embodiment, the second shell 20 is detachably connected to the first shell 18, and the transmission rod 164 is detachably connected to the movable member 14. Furthermore, the constraint member 166 may be fixed in the second shell 20 by engagement or other mechanical fixing manners, such that the aforesaid flange 1664 may be omitted.

In this embodiment, the first shell 18 may form a first chamber C1 for accommodating the drug storage member 10 and the movable member 14, and the second shell 20 may form a second chamber C2 for accommodating the driving mechanism 16. Thus, the second chamber C2 may be detached from the first chamber C1 after injection, as shown in FIG. 10. The first chamber C1 with related components may be disposable after injection, whereas the second chamber C2 with related components may be disposable or reusable after injection.

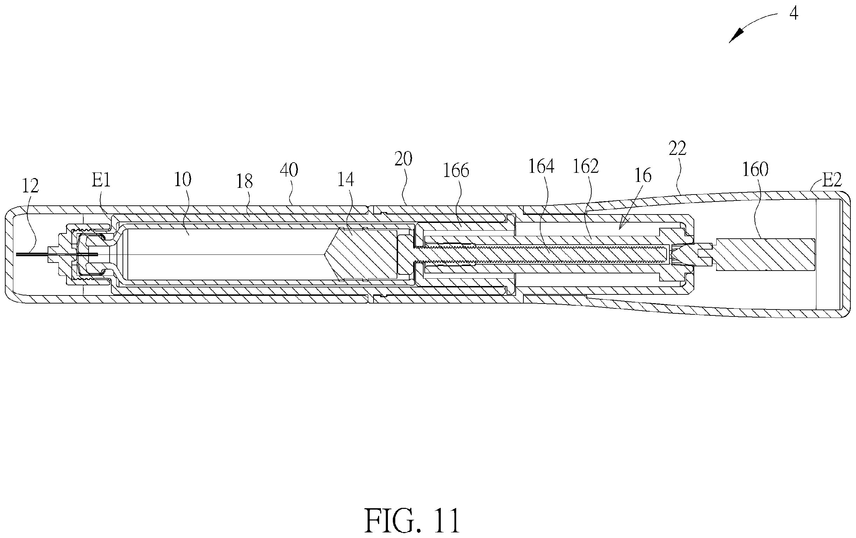

Referring to FIG. 11, FIG. 11 is a sectional view illustrating a tubular injection device 4 according to an embodiment of the invention.

The main difference between the tubular injection device 4 and the aforesaid tubular injection device 2 is that the tubular injection device 4 further comprises a cap 40, as shown in FIG. 11. The cap 40 is configured to cover the first shell 18 and the injection member 12. It should be noted that the cap 40 may also be applied to the aforesaid tubular injection devices 1 and 3.

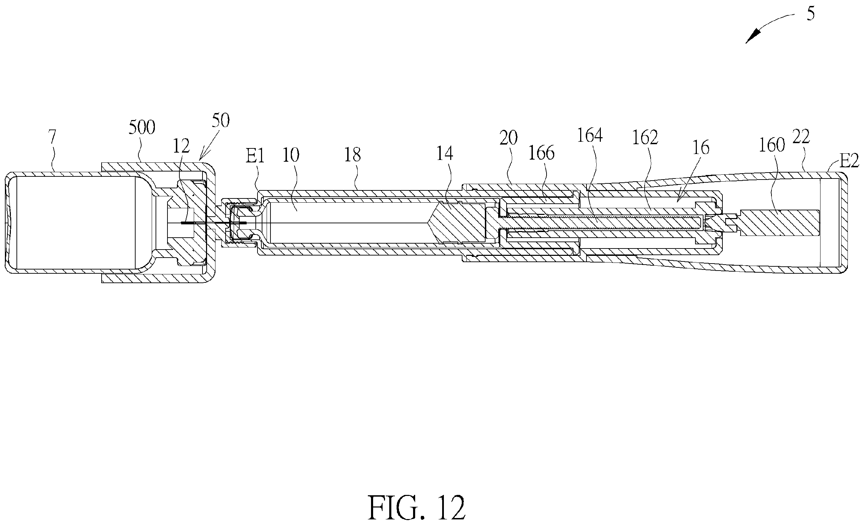

Referring to FIG. 12, FIG. 12 is a sectional view illustrating a tubular injection device 5 according to an embodiment of the invention.

The main difference between the tubular injection device 5 and the aforesaid tubular injection device 2 is that the tubular injection device 5 further comprises an adaptor 50, as shown in FIG. 12. The adaptor 50 is configured to be assembled to the first shell 18. When a user wants to use the tubular injection device 5 to perform an injection process, the user may load a drug container 7 (e.g. vial) in the adaptor 50, as shown in FIG. 12. In this embodiment, the adaptor 50 may comprise a sleeve portion 500 for holding the drug container 7. Furthermore, the sleeve portion 500 may be transparent or contains a window, allowing users to inspect the drug container 7 loaded in the adaptor 50 and a medical fluid inside the drug container 7.

When the drug container 7 is loaded in the adaptor 50, the injection member 12 penetrates into the drug container 7. Then, the user may activate the driving mechanism 16 to drive the movable member 14 to move to transfer the medical fluid from the drug container 7 to the drug storage member 10 through the injection member 12. It should be noted that the adaptor 50 may also be applied to the aforesaid tubular injection devices 1 and 3.

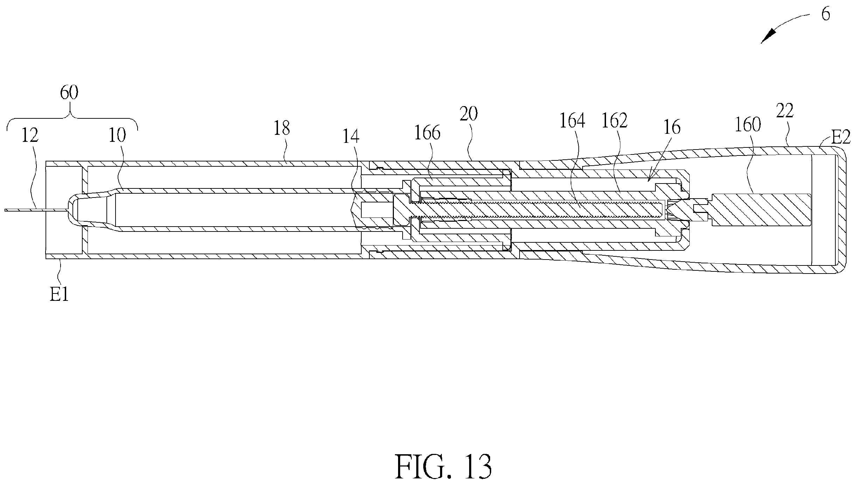

Referring to FIG. 13, FIG. 13 is a sectional view illustrating a tubular injection device 6 according to an embodiment of the invention.

The main difference between the tubular injection device 6 and the aforesaid tubular injection device 2 is that the drug storage member 10 and the injection member 12 of the tubular injection device 6 are combined to form a prefilled syringe 60, as shown in FIG. 13. The injection member 12 extends out of the proximal end E1 of the tubular injection device 6 for purpose of injection. It should be noted that the prefilled syringe 60 may also be applied to the aforesaid tubular injection devices 1 and 3.

As mentioned in the above, the tubular injection device of the invention utilizes the driving mechanism to drive the movable member to move with respect to the drug storage member, so as to achieve automated injection of drug. Thus, users do not need to pull or push the movable member to move by manual operation. Accordingly, the tubular injection device of the invention allows automated injection of drug with minimal manual operation, thereby improving injection operation and ensuring injection safety by minimizing human error. In an embodiment, the tubular injection device of the invention may be equipped with an adaptor for loading a drug container, such that a medical fluid can be transferred from the drug container to the drug storage member automatically by activating the driving mechanism. Thus, the tubular injection device of the invention offers automated operation that replaces manual operation of drug transfer from the drug container to the tubular injection device, which allows self-administration by lay persons.

## Claims

1. A tubular injection device (1, 2, 3, 5) with a proximal end (E1) and a distal end (E2), **characterized by** the tubular injection device (1, 2, 3, 5) comprising:
a drug storage member (10);
an injection member (12) disposed at the proximal end (E1) and being in fluid communication with the drug storage member (10);
a movable member (14) movably disposed in the drug storage member (10); and
a driving mechanism (16) comprising:
a driving member (160) disposed in the distal end (E2);
a transmission sleeve (162) engaged with the driving member (160), the transmission sleeve (162) comprising an internal thread (1620);
a transmission rod (164) disposed in the transmission sleeve (162) and engaged with the movable member (14), the transmission rod (164) comprising an external thread (1640) meshing with the internal thread (1620) of the transmission sleeve (162); and
a constraint member (166) restraining the transmission rod (164) from rotating;
wherein the driving member (160) drives the transmission sleeve (162) to rotate, the transmission sleeve (162) drives the transmission rod (164) to move by an interaction between the internal thread (1620) and the external thread (1640), and the transmission rod (164) drives the movable member (14) to move with respect to the drug storage member (10).

2. The tubular injection device (1, 2, 3, 5) of claim 1 further **characterized in that** when the driving member (160) drives the transmission sleeve (162) to rotate in a first direction (D1), the transmission rod (164) moves linearly toward the proximal end (E1) and drives the movable member (14) to move toward the injection member (12); when the driving member (160) drives the transmission sleeve (162) to rotate in a second direction (D2), the transmission rod (164) drives the movable member (14) to move away from the injection member (12); the first direction (D1) is opposite to the second direction (D2).

3. The tubular injection device (1, 2, 3, 5) of claim 1 or 2 further **characterized in that** the transmission rod (164) has a first limit plane (1642), the constraint member (166) is formed with a through hole (1660), the through hole (1660) has a second limit plane (1662), an end of the transmission rod (164) passes through the through hole (1660), and the constraint member (166) restrains the transmission rod (164) from rotating by the first limit plane (1642) and the second limit plane (1662).

4. The tubular injection device (1, 2, 3, 5) of any of the preceding claims further **characterized in that** the driving mechanism (16) further comprises a screw nut (168) embedded in the transmission sleeve (162), and the internal thread (1620) is provided by the screw nut (168).

5. The tubular injection device (1, 2, 3, 5) of any of the preceding claims further **characterized in that** the tubular injection device (1, 2, 3, 5) further comprises a first shell (18), and the drug storage member (10) is accommodated in the first shell (18).

6. The tubular injection device (1) of claim 5 further **characterized in that** the tubular injection device (1) further comprises a second shell (20), the first shell (18) and the second shell (20) are assembled to accommodate the drug storage member (10) and the driving mechanism (16), the constraint member (166) comprises a flange (1664), and the flange (1664) is sandwiched between the first shell (18) and the second shell (20).

7. The tubular injection device (2, 5) of claim 5 further **characterized in that** the tubular injection device (2, 5) further comprises a second shell (20) and a third shell (22), the first shell (18) and the second shell (20) are assembled to accommodate the drug storage member (10), the transmission sleeve (162), the transmission rod (164) and the constraint member (166), the constraint member (166) comprises a flange (1664), the flange (1664) is sandwiched between the first shell (18) and the second shell (20), the third shell (22) is detachably connected to the second shell (20), the driving member (160) is accommodated in the third shell (22), and the driving member (160) comprises a transmission portion (1600) detachably connected to the transmission sleeve (162).

8. The tubular injection device (3) of claim 5 further **characterized in that** the tubular injection device (3) further comprises a second shell (20), the driving mechanism (16) is accommodated in the second shell (20), the second shell (20) is detachably connected to the first shell (18), and the transmission rod (164) is detachably connected to the movable member (14).

9. The tubular injection device (5) of any of claims 5 to 8 further **characterized in that** the tubular injection device (5) further comprises an adaptor (50) configured to be assembled to the first shell (18); when a drug container (7) is loaded in the adaptor (50), the injection member (12) penetrates into the drug container (7).

10. The tubular injection device (1, 2, 3, 5) of any of the preceding claims further **characterized in that** the injection member (12), the drug storage member (10), the movable member (14) and the driving mechanism (16) are arranged along a longitudinal axis (LA) of the tubular injection device (1, 2, 3, 5) to form a handheld injector.

11. A driving mechanism (16) **characterized by** the driving mechanism (16) comprising:
a driving member (160);
a transmission sleeve (162) engaged with the driving member (160), the transmission sleeve (162) comprising an internal thread (1620);
a transmission rod (164) disposed in the transmission sleeve (162) and engaged with the movable member (14), the transmission rod (164) comprising an external thread (1640) meshing with the internal thread (1620) of the transmission sleeve (162); and
a constraint member (166) restraining the transmission rod (164) from rotating;
wherein the driving member (160) drives the transmission sleeve (162) to rotate, and the transmission sleeve (162) drives the transmission rod (164) to move by an interaction between the internal thread (1620) and the external thread (1640).

12. The driving mechanism (16) of claim 11 further **characterized in that** when the driving member (160) drives the transmission sleeve (162) to rotate in a first direction (D1), the transmission sleeve (162) drives the transmission rod (164) to move out of the transmission sleeve (162); when the driving member (160) drives the transmission sleeve (162) to rotate in a second direction (D2), the transmission sleeve (162) drives the transmission rod (164) to move into the transmission sleeve (162); the first direction (D1) is opposite to the second direction (D2).

13. The driving mechanism (16) of claim 11 or 12 further **characterized in that** the transmission rod (164) has a first limit plane (1642), the constraint member (166) is formed with a through hole (1660), the through hole (1660) has a second limit plane (1662), an end of the transmission rod (164) passes through the through hole (1660), and the constraint member (166) restrains the transmission rod (164) from rotating by the first limit plane (1642) and the second limit plane (1662).

14. The driving mechanism (16) of any of the preceding claims further **characterized in that** the driving mechanism (16) further comprises a screw nut embedded in the transmission sleeve (162), and the internal thread (1620) is provided by the screw nut.

15. The driving mechanism (16) of any of the preceding claims further **characterized in that** the driving member (160) comprises a transmission portion (1600) detachably engaged with the transmission sleeve (162).

## Amended claims

### Amended claims in accordance with Rule 137(2) EPC.

1. A driving mechanism (16) comprising:
a driving member (160);
a transmission sleeve (162) engaged with the driving member (160), the transmission sleeve (162) comprising an internal thread (1620);
a transmission rod (164) disposed in the transmission sleeve (162), the transmission rod (164) comprising an external thread (1640) meshing with the internal thread (1620) of the transmission sleeve (162); and
a constraint member (166) restraining the transmission rod (164) from rotating;wherein the driving member (160) drives the transmission sleeve (162) to rotate, and the transmission sleeve (162) drives the transmission rod (164) to move by an interaction between the internal thread (1620) and the external thread (1640);
**characterized by**
wherein, when the driving member (160) drives the transmission sleeve (162) to rotate in a first direction (D1), the transmission sleeve (162) drives the transmission rod (164) to move out of the transmission sleeve (162);
wherein, when the driving member (160) drives the transmission sleeve (162) to rotate in a second direction (D2), the transmission sleeve (162) drives the transmission rod (164) to move into the transmission sleeve (162); the first direction (D1) is opposite to the second direction (D2).

2. The driving mechanism (16) of claim 1 further **characterized in that** the transmission rod (164) has a first limit plane (1642), the constraint member (166) is formed with a through hole (1660), the through hole (1660) has a second limit plane (1662), an end of the transmission rod (164) passes through the through hole (1660), and the constraint member (166) restrains the transmission rod (164) from rotating by the first limit plane (1642) and the second limit plane (1662).

3. The driving mechanism (16) of any of the preceding claims further **characterized in that** the driving mechanism (16) further comprises a screw nut (168) embedded in the transmission sleeve (162), and the internal thread (1620) is provided by the screw nut (168).

4. The driving mechanism (16) of any of the preceding claims further **characterized in that** the driving member (160) comprises a transmission portion (1600) detachably engaged with the transmission sleeve (162).

5. A tubular injection device (1, 2, 3, 5) with a proximal end (E1) and a distal end (E2) comprising:
a drug storage member (10);
an injection member (12) disposed at the proximal end (E1) and being in fluid communication with the drug storage member (10);
a movable member (14) movably disposed in the drug storage member (10); and
**characterized by** the tubular injection device (1, 2, 3, 5) further comprising a driving mechanism (16) of any of claims 1 to 3;
wherein the driving member (160) is disposed in the distal end (E2), the transmission rod (164) is engaged with the movable member (14), and the transmission rod (164) drives the movable member (14) to move with respect to the drug storage member (10)

6. The tubular injection device (1, 2, 3, 5) of claim 5 further **characterized in that** the tubular injection device (1, 2, 3, 5) further comprises a first shell (18), and the drug storage member (10) is accommodated in the first shell (18).

7. The tubular injection device (1) of claim 6 further **characterized in that** the tubular injection device (1) further comprises a second shell (20), the first shell (18) and the second shell (20) are assembled to accommodate the drug storage member (10) and the driving mechanism (16), the constraint member (166) comprises a flange (1664), and the flange (1664) is sandwiched between the first shell (18) and the second shell (20).

8. The tubular injection device (2, 5) of claim 6 further **characterized in that** the tubular injection device (2, 5) further comprises a second shell (20) and a third shell (22), the first shell (18) and the second shell (20) are assembled to accommodate the drug storage member (10), the transmission sleeve (162), the transmission rod (164) and the constraint member (166), the constraint member (166) comprises a flange (1664), the flange (1664) is sandwiched between the first shell (18) and the second shell (20), the third shell (22) is detachably connected to the second shell (20), the driving member (160) is accommodated in the third shell (22), and the driving member (160) comprises a transmission portion (1600) detachably connected to the transmission sleeve (162).

9. The tubular injection device (3) of claim 6 further **characterized in that** the tubular injection device (3) further comprises a second shell (20), the driving mechanism (16) is accommodated in the second shell (20), the second shell (20) is detachably connected to the first shell (18), and the transmission rod (164) is detachably connected to the movable member (14).

10. The tubular injection device (5) of any of claims 6 to 9 further **characterized in that** the tubular injection device (5) further comprises an adaptor (50) configured to be assembled to the first shell (18); when a drug container (7) is loaded in the adaptor (50), the injection member (12) penetrates into the drug container (7).

11. The tubular injection device (1, 2, 3, 5) of any of claims 5 to 10 further **characterized in that** the injection member (12), the drug storage member (10), the movable member (14) and the driving mechanism (16) are arranged along a longitudinal axis (LA) of the tubular injection device (1, 2, 3, 5) to form a handheld injector.
